# EUROPEAN PATENT APPLICATION

(11) **EP 4 792 729 A1**
(43) Date of publication of application: **19.08.2026**
(21) Application number: 24877121.4
(22) Date of filing: 04.10.2024
(51) Int. Cl.: A61B 1/24, A61B 1/00, A61B 1/045

(54) **IMAGE PROCESSING METHOD, IMAGE PROCESSING DEVICE, AND PROGRAM**

(30) Priority: 12.10.2023 JP 2023177074
(71) Applicant: Panasonic Intellectual Property Management Co., Ltd., Kadoma-shi, Osaka 571-0057 (JP)
(72) Inventor: HAMASAKI, Takeshi, Kadoma-shi, Osaka 571-0057 (JP); OHTSUKA, Yoshio, Kadoma-shi, Osaka 571-0057 (JP); MINAHASHI, Kan, Kadoma-shi, Osaka 571-0057 (JP)
(74) Representative: Novagraaf International SA
(86) International application number: PCT/JP2024/035616
(87) International publication number: WO 2025/079525

(57) **Abstract**

An image processing method is an image processing method for displaying a dental plaque region based on an image showing a tooth and dental plaque fluorescing in response to exposure to light including a blue-light wavelength range. The image processing method includes: detecting a natural tooth region from a region showing the tooth, based on the image (S201 to S206); detecting a dental plaque region based on the image (S207); and displaying the dental plaque region restricted to the natural tooth region (S105). For instance, in the image processing method, the detecting of the natural tooth region may include detecting a first region that is (i) a region with a luminance value greater than or equal to a predetermined first threshold within an all-pixel region in the image or (ii) a region with a green pixel value greater than or equal to a predetermined second threshold within the all-pixel region in the image, to detect the natural tooth region based on the first region.

## Description

### [Technical Field]

The present disclosure relates to an image processing method, an image processing device, and a program.

### [Background Art]

Devices that detect dental plaque based on an image showing teeth inside an oral cavity are known. For instance, Patent Literature (PTL) 1 discloses a technique for storing a care region within an oral cavity and the dental plaque amount detected for the care region. Moreover, PTL 2 discloses a technique for correcting a dental image.

### [Citation List]

### [Patent Literature]

[PTL 1] Japanese Unexamined Patent Application Publication No. 2013-042906
[PTL 2] Japanese Unexamined Patent Application Publication No. 2009-37414

### [Summary of Invention]

### [Technical Problem]

There is a need to improve the accuracy of detecting dental plaque in such devices that detect dental plaque.

In view of this, the present disclosure provides an image processing method or an image processing device capable of improving the detection accuracy of dental plaque.

### [Solution to Problem]

An image processing method according to one aspect of the present disclosure is an image processing method for displaying a dental plaque region based on an image showing a tooth and dental plaque fluorescing in response to exposure to light including a blue-light wavelength range. The image processing method includes: detecting a natural tooth region from a region showing the tooth, based on the image; detecting a dental plaque region based on the image; and displaying the dental plaque region restricted to the natural tooth region.

### [Advantageous Effects of Invention]

The present disclosure can provide an image processing method or an image processing device capable of improving the detection accuracy of dental plaque.

### [Brief Description of Drawings]

[FIG. 1] FIG. 1 is a perspective view of an intraoral camera of an intraoral camera system according to an embodiment.
[FIG. 2] FIG. 2 is a cross-sectional view schematically illustrating an imaging optical system incorporated into the intraoral camera of the intraoral camera system according to the embodiment.
[FIG. 3] FIG. 3 illustrates a schematic configuration of the intraoral camera system according to the embodiment.
[FIG. 4] FIG. 4 illustrates a procedure of an operation of the intraoral camera system according to the embodiment.
[FIG. 5]

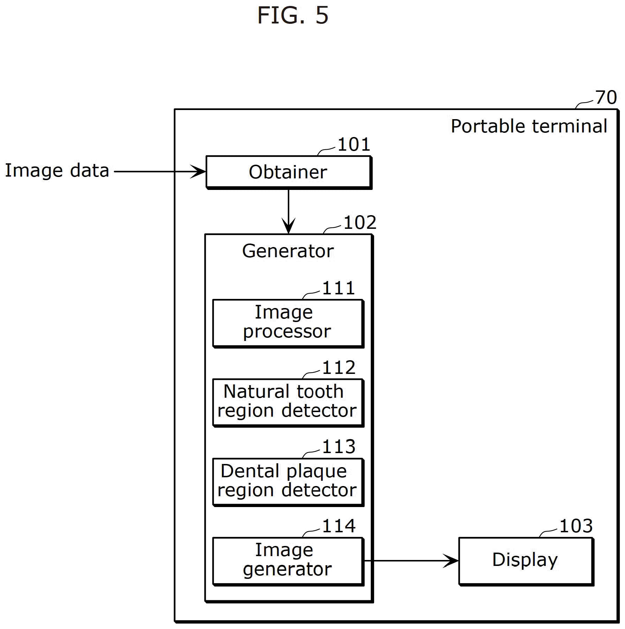

   FIG. 5 is a functional block diagram of a portable terminal according to the embodiment.
[FIG. 6]

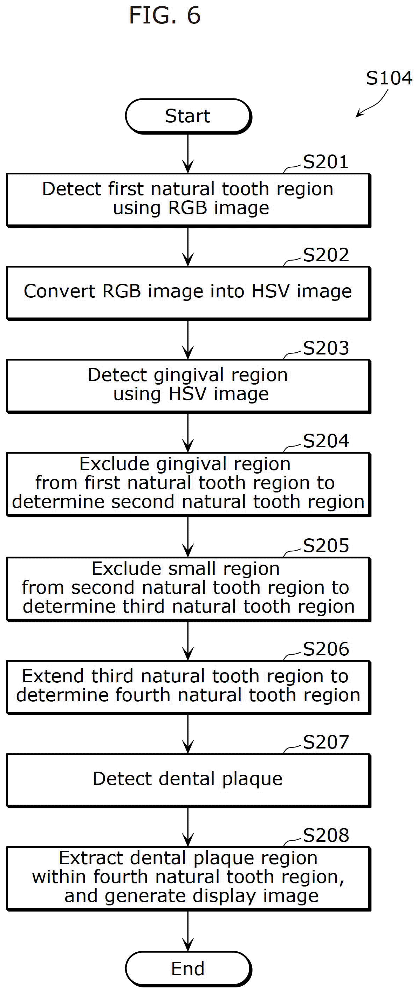

   FIG. 6 is a flowchart of image generation processing according to the embodiment.
[FIG. 7]

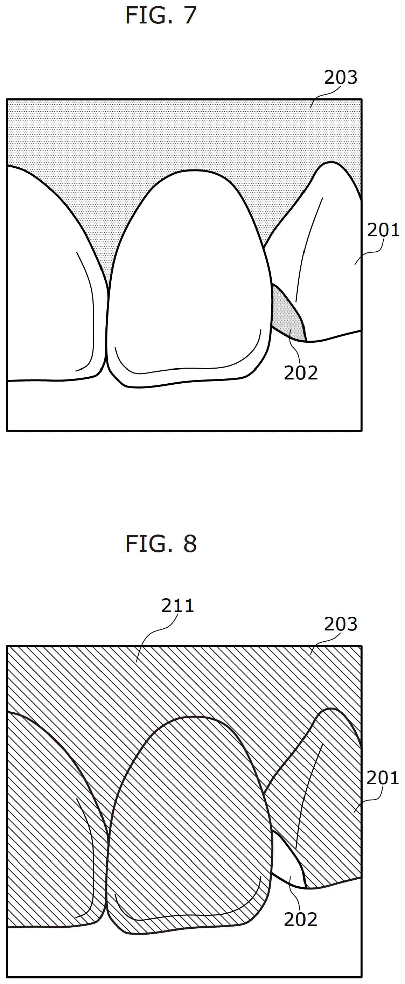

   FIG. 7 illustrates an example of an RGB image (a fourth RGB image) according to the embodiment.
[FIG. 8]
   FIG. 8 illustrates an example of a first natural tooth region according to the embodiment.

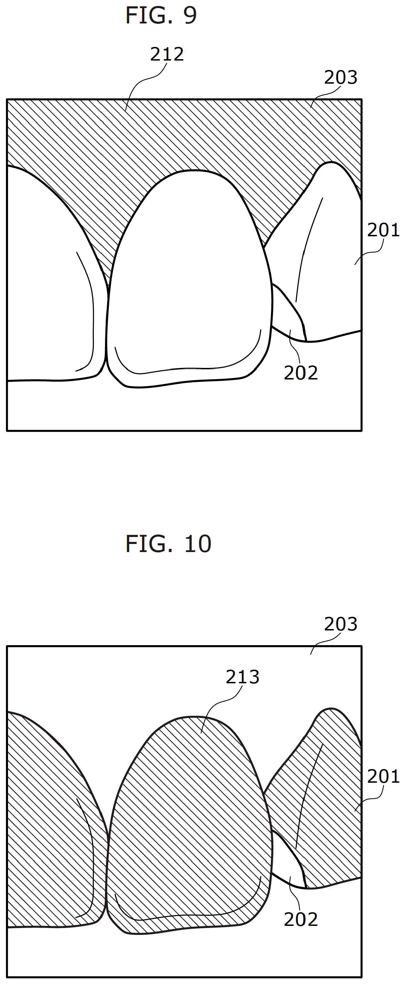

[FIG. 9] FIG. 9 illustrates an example of a gingival region according to the embodiment.
[FIG. 10] FIG. 10 illustrates an example of a second natural tooth region according to the embodiment.

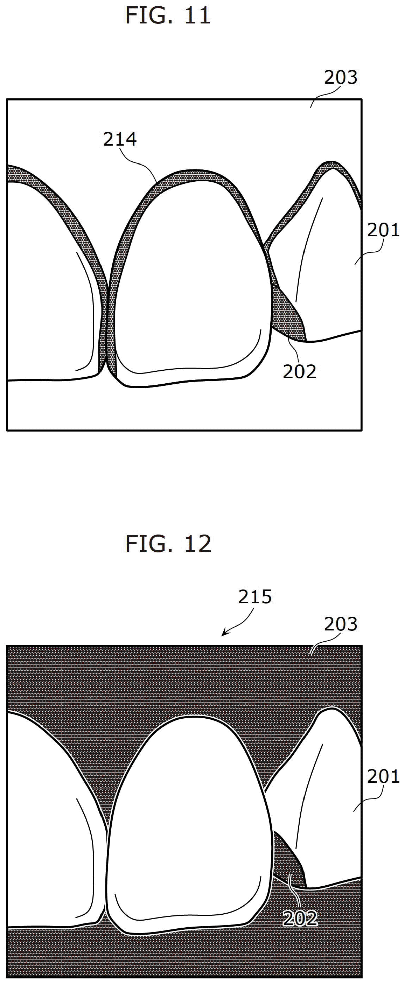

[FIG. 11] FIG. 11 illustrates an example of a first dental plaque region according to the embodiment.
[FIG. 12] FIG. 12 illustrates an example of a mask image according to the embodiment.

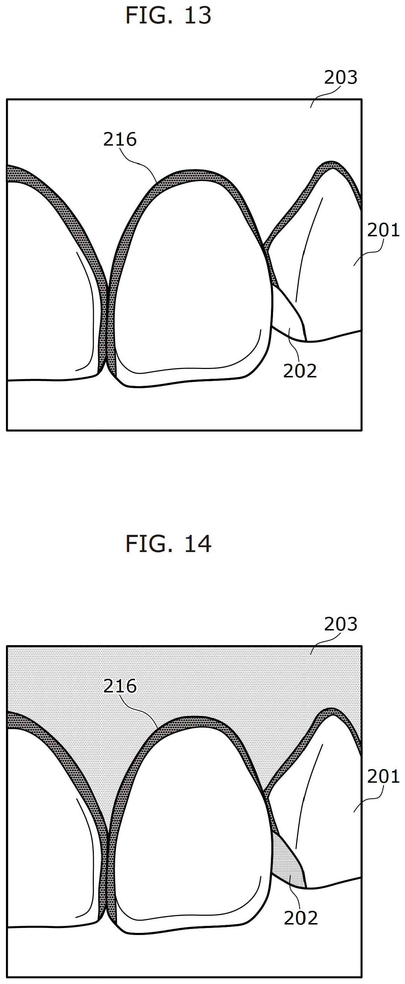

[FIG. 13] FIG. 13 illustrates an example of a second tooth region according to the embodiment.
[FIG. 14] FIG. 14 illustrates an example of a display image according to the embodiment.

### [Description of Embodiments]

When an oral cavity is irradiated with blue light for detection of dental plaque, the blue light is reflected off the surface of a prosthesis made of, for example, gold or silver. This may lead to a false detection of red-wavelength light contained in the blue light as red fluorescence from dental plaque. Moreover, when a user captures an image of a tooth surface with a handheld intraoral camera, it is difficult to distinguish between red fluorescence from dental plaque and reflected light from a prosthesis surface.

In view of this, the present disclosure detects a natural tooth region, obtained by excluding from a tooth region an artificial tooth region, such as a prosthesis, which enables dental plaque detection only within the natural tooth region. This can prevent a false detection of dental plaque due to a prosthesis or the like.

An image processing method according to one aspect of the present disclosure is an image processing method for displaying a dental plaque region based on an image showing a tooth and dental plaque fluorescing in response to exposure to light including a blue-light wavelength range. The image processing method includes: detecting a natural tooth region from a region showing the tooth, based on the image; detecting a dental plaque region based on the image; and displaying the dental plaque region restricted to the natural tooth region.

Thus, the image processing method can suppress false detection of a prosthesis or the like as dental plaque. Thus, the image processing method can improve the detection accuracy of dental plaque.

For instance, in the image processing method, the detecting of the natural tooth region may include: detecting a first region that is (i) a region with a luminance value greater than or equal to a predetermined first threshold within an all-pixel region in the image or (ii) a region with a green pixel value greater than or equal to a predetermined second threshold within the all-pixel region in the image, to detect the natural tooth region based on the first region.

Thus, the image processing method can detect the natural tooth region with high accuracy by using the luminance value or the green pixel value.

For instance, in the image processing method, the detecting of the natural tooth region may include: detecting, as the natural tooth region, a region which is included in the first region and in which each of pairwise differences between a green pixel value, a red pixel value, and a blue pixel value is less than a predetermined third threshold.

Thus, the image processing method can detect the natural tooth region with high accuracy by using the pairwise differences between the green pixel value, the red pixel value, and the blue pixel value.

For instance, in the image processing method, the detecting of the natural tooth region may include: detecting a gingival region based on a hue of the image; and detecting, as the natural tooth region, a region obtained by excluding the gingival region from the first region.

Thus, the image processing method can suppress false determination of the gingival region as the natural tooth region, which, in turn, can improve the detection accuracy of the natural tooth region.

For instance, in the image processing method, the detecting of the natural tooth region may include: detecting, as the natural tooth region, a region obtained by excluding a region having an area smaller than a predetermined area from the first region.

Thus, the image processing method can suppress falsely determining as the natural tooth region, for example, a region with a high luminance due to reflected light or the like in a portion other than the natural tooth region, which, in turn, can improve the detection accuracy of the natural tooth region.

For instance, in the image processing method, the detecting of the natural tooth region may include: detecting, as the natural tooth region, a region obtained by extending a boundary of the first region outward by a predetermined amount.

Thus, by extending the natural tooth region, the image processing method can prevent the dental plaque region from being excessively omitted from display.

Moreover, an image processing device according to another aspect of the present disclosure is an image processing device that displays a dental plaque region based on an image showing a tooth and dental plaque fluorescing in response to exposure to light including a blue-light wavelength range. The image processing device includes: a natural tooth region detector that detects a natural tooth region from a region showing the tooth, based on the image; a dental plaque region detector that detects a dental plaque region based on the image; and a display that displays the dental plaque region restricted to the natural tooth region.

Thus, the image processing device can suppress false detection of a prosthesis or the like as dental plaque. Accordingly, the image processing device can improve the detection accuracy of dental plaque.

Moreover, a program according to one aspect of the present disclosure is a program for causing a computer to execute the above image processing method.

It should be noted that these general or specific aspects may be implemented as a system, a method, an integrated circuit, a computer program, or a computer-readable recording medium, such as a CD-ROM, or may be implemented by any combination of the system, method, integrated circuit, computer program, and recording medium.

Hereinafter, an embodiment is described in detail with reference to the drawings as necessary. However, redundant explanations may be omitted. For instance, detailed explanations for well-known matters and overlapping explanations for substantially the same elements may be omitted. This is to avoid redundant explanations and facilitate understanding by those skilled in the art.

It should be noted that the inventors provide the appended drawings and the explanations below in order to help those skilled in the art to fully understand the present disclosure. Thus, the drawings and the explanations do not intend to limit the subject matter recited in the claims.

### (Embodiment)

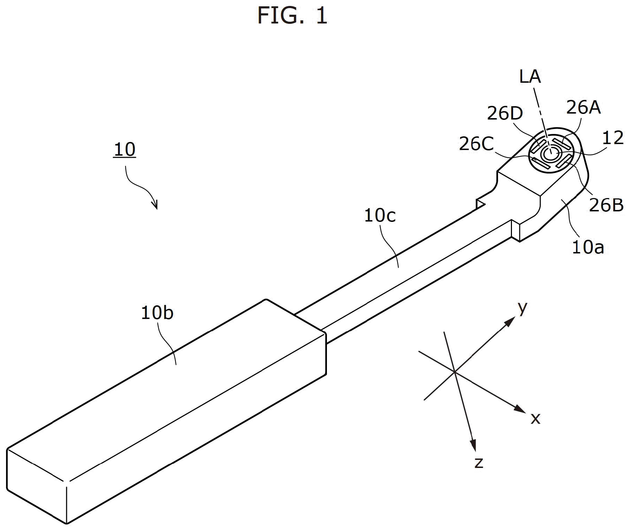

FIG. 1 is a perspective view of an intraoral camera of an intraoral camera system according to an embodiment. As illustrated in FIG. 1, intraoral camera 10 includes a toothbrush-shaped case that can be handled by one hand. The case includes head 10a, handle 10b, and neck 10c. Head 10a is put inside the oral cavity of a user when a dentition image is captured. Handle 10b is configured to be held by the user. Neck 10c connects head 10a and handle 10b.

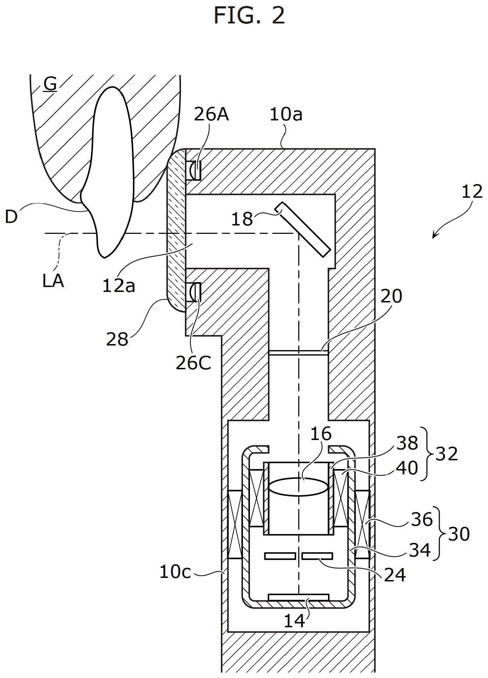

FIG. 2 is a cross-sectional view schematically illustrating imaging optical system 12 incorporated into intraoral camera 10. As illustrated in FIG. 2, for the embodiment, imaging optical system 12 of intraoral camera 10 is incorporated into head 10a and neck 10c. Imaging optical system 12 includes image sensor 14 and lens 16 disposed on optical axis LA thereof.

Image sensor 14 is an imaging device, such as a complementary metal-oxide-semiconductor (CMOS) sensor or a charge-coupled device (CCD), and lens 16 forms an image of tooth D. Image sensor 14 outputs a signal (image data) corresponding to the formed image to an external destination.

Lens 16 is, for example, a condenser lens, and when light from tooth D is incident on lens 16, lens 16 forms an image of tooth D onto image sensor 14. It should be noted that lens 16 may be one lens or a lens group including a plurality of lenses.

For the embodiment, imaging optical system 12 further includes mirror 18, blue-light blocking filter (blue-light blocking element) 20, and aperture 24. Mirror 18 reflects the image of tooth D toward lens 16. Blue-light blocking filter 20 is disposed between mirror 18 and lens 16. Aperture 24 is disposed between lens 16 and image sensor 14.

Mirror 18 is disposed on optical axis LA of imaging optical system 12 so as to reflect the image of tooth D which has passed through light entry port 12a of imaging optical system 12 toward lens 16.

Blue-light blocking filter 20 is a filter that blocks a blue-wavelength light component included in light to be incident on image sensor 14. When teeth are irradiated with light including a blue-light wavelength range to detect dental plaque, as the light including the blue-light wavelength range is intensified to intensify the excitation fluorescence of the dental plaque, the entirety of a first RGB image takes on a blue tinge. In this state, a blue pixel value is dominant over a red pixel value and a green pixel value, which may decrease the effect of making it easier to identify a dental plaque region by performing image processing (exposure control processing and white balance adjustment processing), which is described later. To deal with this, blue-light blocking filter 20 blocks, from light yet to enter image sensor 14, a portion of the light including the blue-light wavelength range.

Aperture 24 is a plate-like component having a through hole on optical axis LA of imaging optical system 12 and achieves a deep depth of focus. Thus, a point along the depth direction within an oral cavity can be brought into focus, and a dentition image with a clear outline can be obtained.

Moreover, intraoral camera 10 includes LEDs 26A to 26D as illumination devices that irradiate target tooth D with light during image capturing. LEDs 26A to 26D are, for example, blue light-emitting diodes (LEDs). Moreover, as illustrated in FIG. 1, for the embodiment, LEDs 26A to 26D surround light entry port 12a. It should be noted that translucent cover 28 covering LEDs 26A to 26D and light entry port 12a is provided on head 10a to avoid insufficient illumination caused by gums G and so forth coming into contact with LEDs 26A to 26D. It should be noted that one or more of LEDs 26A to 26D may be one or more white LEDs. By using white LEDs as some of LEDs 26A to 26D, it is possible to increase the brightness of the first RGB image, which can improve the balance between the blue pixel value and the red pixel value and the balance between the blue pixel value and the green pixel value.

Furthermore, for the embodiment, as illustrated in FIG. 2, intraoral camera 10 includes composition adjustment mechanism 30 and focus adjustment mechanism 32.

Composition adjustment mechanism 30 includes case 34 holding image sensor 14 and lens 16 and actuator 36 for moving case 34 in a direction in which optical axis LA extends. When actuator 36 adjusts the position of case 34, the angle of view is adjusted, that is, the size of a dentition whose image is to be formed onto image sensor 14 is adjusted. It should be noted that composition adjustment mechanism 30 automatically adjusts the position of case 34 such that the entirety of a tooth is included in a captured image, for example. Moreover, in accordance with a user operation, composition adjustment mechanism 30 adjusts the position of case 34 to achieve the angle of view desired by the user.

Focus adjustment mechanism 32 is held inside case 34 of composition adjustment mechanism 30, and includes lens holder 38 holding lens 16 and actuator 40 for moving lens holder 38 in the direction in which optical axis LA extends. The focus is adjusted by actuator 40 adjusting the relative position of lens holder 38 relative to image sensor 14. It should be noted that focus adjustment mechanism 32 automatically adjusts the position of lens holder 38 such that a tooth positioned in the middle of an image being captured is brought into focus, for example. Moreover, focus adjustment mechanism 32 adjusts the position of lens holder 38 in accordance with a user operation.

It should be noted that the elements of imaging optical system 12 except for mirror 18 may be provided in handle 10b of intraoral camera 10.

The image output by image sensor 14 is an RGB image in which each of the pixels of the image includes RGB subpixels.

Moreover, intraoral camera 10 includes LEDs 26A to 26D as illumination devices that irradiate a target tooth with light during image capturing. LEDs 26A to 26D are, for example, blue LEDs that emit blue light having a wavelength peak of 405 nm. It should be noted that LEDs 26A to 26D may be light sources for emitting light including the blue-light wavelength range and are not limited to blue LEDs.

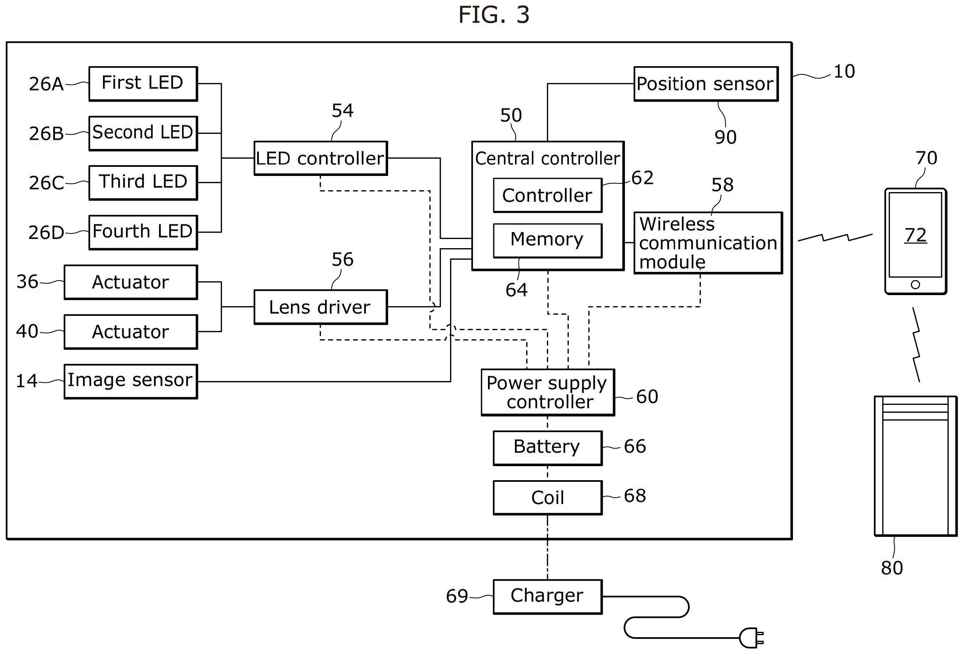

FIG. 3 illustrates a schematic configuration of the intraoral camera system according to the embodiment. As illustrated in FIG. 3, in overview, the intraoral camera system according to the embodiment is configured to capture a dentition image by using intraoral camera 10 and perform the image processing on the captured image.

As illustrated in FIG. 3, the intraoral camera system includes intraoral camera 10, portable terminal 70, and cloud server 80. Portable terminal 70 is, for example, a smartphone or a tablet terminal capable of performing wireless communication. Portable terminal 70 includes, as an input device and an output device, touch screen 72 capable of displaying, for example, the dentition image. Portable terminal 70 functions as a user interface of the intraoral camera system.

Cloud server 80 is a server capable of communicating with portable terminal 70 via, for example, the Internet and provides portable terminal 70 with an application for using intraoral camera 10. For instance, the user downloads the application from cloud server 80 and installs the application on portable terminal 70. Moreover, cloud server 80 obtains, via portable terminal 70, the dentition image captured by intraoral camera 10.

Intraoral camera 10 includes, as main parts that perform system control, central controller 50, LED controller 54, lens driver 56, and position sensor 90. Here, LED controller 54 controls LEDs 26A to 26D, and lens driver 56 controls actuator 36 of composition adjustment mechanism 30 and actuator 40 of focus adjustment mechanism 32.

Moreover, intraoral camera 10 includes wireless communication module 58 that performs wireless communication with portable terminal 70 and power supply controller 60 that supplies power to, for example, central controller 50.

Central controller 50 of intraoral camera 10 is included in, for example, handle 10b of intraoral camera 10. Moreover, for instance, central controller 50 includes controller 62, such as a central processing unit (CPU) or a micro processing unit (MPU), which performs various processing tasks described later, and memory 64, such as random access memory (RAM) or read only memory (ROM), storing a program for causing controller 62 to perform the various processing tasks. It should be noted that memory 64 stores, for example, a dentition image (image data) captured by image sensor 14 and various setting data items, in addition to the program. The dentition image captured by image sensor 14 is an example of the first RGB image.

Controller 62 transmits the dentition image output by image sensor 14 to portable terminal 70 via wireless communication module 58. Portable terminal 70 displays the transmitted dentition image on touch screen 72 and thus presents the dentition image to the user.

LED controller 54 is included in, for example, handle 10b of intraoral camera 10, and switches on and off LEDs 26A to 26D in accordance with a control signal from controller 62. LED controller 54 is, for example, a circuit. When for instance the user performs an operation to activate intraoral camera 10 on touch screen 72 of portable terminal 70, a corresponding signal is transmitted from portable terminal 70 to controller 62 via wireless communication module 58. In accordance with the received signal, controller 62 transmits a control signal to LED controller 54 to cause LED controller 54 to switch on LEDs 26A to 26D.

Lens driver 56 is included in, for example, handle 10b of intraoral camera 10, and controls actuator 36 of composition adjustment mechanism 30 and actuator 40 of focus adjustment mechanism 32 in accordance with a control signal from controller 62 of central controller 50. Lens driver 56 is, for example, a circuit. When for instance the user performs an operation related to composition adjustment or focus adjustment on touch screen 72 of portable terminal 70, a corresponding signal is transmitted from portable terminal 70 to central controller 50 via wireless communication module 58. In accordance with the received signal, controller 62 of central controller 50 transmits a control signal to lens driver 56 to cause lens driver 56 to perform the composition adjustment or the focus adjustment. Moreover, for instance, in accordance with the dentition image received from image sensor 14, controller 62 calculates the amount of control for actuator 36 or actuator 40 required for the composition adjustment or the focus adjustment. Then, a control signal corresponding to the calculated amount of control is transmitted to lens driver 56.

Wireless communication module 58 is included in, for example, handle 10b of intraoral camera 10, and performs wireless communication with portable terminal 70 in accordance with a control signal from controller 62. Wireless communication module 58 performs, with portable terminal 70, wireless communication complying with an existing communication standard, such as Wi-Fi (registered trademark) or Bluetooth (registered trademark). A dentition image showing tooth D is transmitted from intraoral camera 10 to portable terminal 70 via wireless communication module 58, and an operation signal is transmitted from portable terminal 70 to intraoral camera 10 via wireless communication module 58.

For the embodiment, power supply controller 60 is included in handle 10b of intraoral camera 10, and distributes the power of battery 66 to central controller 50, LED controller 54, lens driver 56, and wireless communication module 58. Power supply controller 60 is, for example, a circuit. It should be noted that, for the embodiment, battery 66 is a rechargeable (secondary) battery, and is wirelessly charged by external charger 69 connected to a commercial power source, via coil 68 included in intraoral camera 10.

Position sensor 90 is a sensor for detecting the orientation and the position of intraoral camera 10 and, for example, a multi-axis (here, X, Y, and Z-axis, that is, three-axis) acceleration sensor. For instance, position sensor 90 may be a six-axis sensor including a three-axis acceleration sensor and a three-axis gyro sensor. For instance, as illustrated in FIG. 1, the Z-axis matches optical axis LA. The Y-axis is parallel to the imaging plane and extends in a longitudinal direction of intraoral camera 10. Moreover, the X-axis is parallel to the imaging plane and orthogonal to the Y-axis. Output by position sensor 90 for each axis may be transmitted to portable terminal 70 via central controller 50 and wireless communication module 58.

A piezo-resistive type, capacitive type, or heat detection type micro-electro-mechanical systems (MEMS) sensor may be used as position sensor 90. Although not illustrated in the figures, a correction circuit for correcting, for example, the balance of sensor sensitivity between the axes, the temperature characteristics of sensitivity, or temperature drift may be provided. Moreover, a bandpass filter (a low pass filter) for removing dynamic acceleration components or noise may be provided. Moreover, noise may be reduced by smoothing a waveform output by the acceleration sensor.

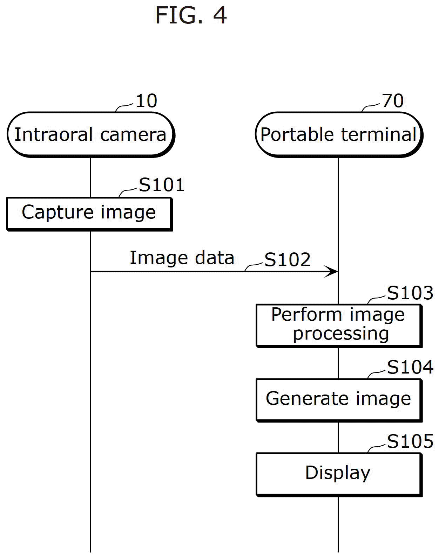

Then, an operation of the intraoral camera system is described. FIG. 4 illustrates a procedure of an operation of the intraoral camera system. It should be noted that the processing illustrated in FIG. 4 is, for example, processing performed in real time, and is performed each time image data including one or more frames is obtained.

Image data is generated by the user capturing an image of teeth and gums inside their oral cavity with intraoral camera 10 (S101). The image data is, for example, image data obtained by imaging teeth fluorescing in response to exposure to light including a blue-light wavelength range. Then, intraoral camera 10 transmits the captured image data to portable terminal 70 (S102). It should be noted that the image data described here may be a video or one or more still images. Moreover, when the image data is a video or includes still images, sensor data may be transmitted for each frame of the video or each still image. It should be noted that when the image data is a video, sensor data may be transmitted every two or more frames.

Moreover, the image data may be transmitted in real time or transmitted at once after a series of imaging (e.g., after imaging all the teeth inside the oral cavity).

Portable terminal 70 performs the image processing on the received image data (S103), generates an image showing a dental plaque region by using the image data after the image processing (S104), and displays the generated image (S105).

By using such an intraoral camera system, the user can capture an intraoral image of the user with intraoral camera 10 and check the intraoral condition displayed on portable terminal 70. Furthermore, from a dental plaque region shown in the displayed image, the user can readily check for any missed spots in brushing.

Moreover, portable terminal 70 may, for instance, generate the three-dimensional models of teeth inside the oral cavity from captured image data items. Moreover, portable terminal 70 may display an image based on the generated three-dimensional models.

It should be noted that an example in which portable terminal 70 performs processing on a tooth image is described here. However, intraoral camera 10 may perform part of the processing or the whole processing. Portable terminal 70 is an example of an image processing device (a dental plaque detection device).

FIG. 5 is a functional block diagram of portable terminal 70. Portable terminal 70 includes obtainer 101, generator 102, and display 103.

Obtainer 101 obtains image data (a first RGB image) transmitted from intraoral camera 10. Obtainer 101 may obtain sensor data in addition to the image data from intraoral camera 10. The first RGB image is an image obtained by intraoral camera 10 imaging teeth fluorescing in response to exposure to light including a blue-light wavelength range. Here, blue light is an example of irradiation light of a predetermined wavelength that excites a fluorescent substance contained in dental plaque. Moreover, the fluorescent substance is, for example, porphyrin.

Generator 102 generates a display image showing a dental plaque region, using the image data (the first RGB image) obtained by obtainer 101. Generator 102 includes image processor 111, natural tooth region detector 112, dental plaque region detector 113, and image generator 114.

Image processor 111 performs image processing on the first RGB image as preprocessing. Specifically, image processor 111 performs inverse gamma conversion (inverse gamma correction) on the first RGB image, to generate a second RGB image. Next, image processor 111 performs white balance adjustment processing on the second RGB image, to generate a third RGB image. Then, image processor 111 performs gamma conversion (gamma correction) on the third RGB image, to generate a fourth RGB image.

The white balance adjustment processing is processing for adjusting, for example, the tone of white in the image. For instance, image processor 111 extracts pixels having RGB values satisfying the following Expressions 1 and 2, from a plurality of second RGB pixels making up the second RGB image to be processed.$min \left(R, G, B\right) \leq Ths , and max \left(R, G, B\right) < Thmax$$Thl \leq Y \leq Thu$

In Expression 1, the smallest value among the pixel values of the three RGB subpixels of a second RGB pixel (that is, a red pixel value, a green pixel value, and a blue pixel value) is indicated by min(R, G, B).

Ths is a threshold for excluding a region (e.g., a glossy region) strongly affected by reflection of irradiation light in the first RGB image. Ths is, for example, 900 in the 10-bit representation.

The largest value among the pixel values of the three RGB subpixels of the second RGB pixel (that is, the red pixel value, the green pixel value, and the blue pixel value) is indicated by max(R, G, B).

Thmax indicates the largest value that the pixel values can take. Thmax is expressed as, for example, 1023 in the 10-bit representation.

In Expression 2, Thl is a threshold indicating the lower limit of a tooth region, and Thu is a threshold indicating the upper limit of the tooth region.

The tooth region in the second RGB image is extracted using Expression 2. That is, a plurality of pixels extracted using Expressions 1 and 2 are a plurality of pixels making up the tooth region.

Then, image processor 111 calculates first red pixel average value Rave that is the average value of red pixel values in the tooth region satisfying Expressions 1 and 2, first green pixel average value Gave that is the average value of green pixel values in the tooth region, and first blue pixel average value Bave that is the average value of blue pixel values in the tooth region. Then, image processor 111 adjusts a gain for each of at least two color components among the red component, the green component, and the blue component of an RGB image to be processed, to equalize first red pixel average value Rave, first green pixel average value Gave, and first blue pixel average value Bave.

Specifically, image processor 111 calculates the gain for the red pixel values (the gain for red pixels) by dividing first green pixel average value Gave by first red pixel average value Rave. Moreover, image processor 111 calculates the gain for the blue pixel values (the gain for blue pixels) by dividing first green pixel average value Gave by first blue pixel average value Bave. Then, image processor 111 generates a third RGB image by multiplying each of the red pixels of the plurality of second RGB pixels making up the second RGB image by the gain for the red pixels and multiplying each of the blue pixels of the plurality of second RGB pixels by the gain for the blue pixels. In other words, the pixel values of a plurality of third RGB pixels making up the third RGB image are pixel values obtained by multiplying the red pixel values of the plurality of second RGB pixels making up the second RGB image by the gain for the red pixels and multiplying the blue pixel values of the plurality of second RGB pixels by the gain for the blue pixels. It should be noted that in the above example, image processor 111 performs the white balance adjustment processing by calculating the gain for the red pixels and the gain for the blue pixels by using the green pixel average value as a reference and multiplying, by each gain, the pixel values of a color component corresponding to the gain. However, using the green pixel average value as the reference is just an example. The gain for the green pixels and the gain for the blue pixels may be calculated using the red pixel average value as the reference. The gain for the red pixels and the gain for the green pixels may be calculated using the blue pixel average value as the reference.

It should be noted that if a pixel value multiplied by the gain exceeds the largest value (1023 for the 10-bit representation), image processor 111 replaces the pixel value with 1023.

It should be noted that the white balance adjustment processing illustrated herein is just an example, and other methods may be used. Moreover, the details of the image processing described herein are just an example, and other processing may be used. For instance, the image processing may include exposure control processing. Moreover, among the processes described above, only some processes may be performed, and the image processing need not be performed. That is, for example, natural tooth region detection processing, which is described later, may be performed using the first RGB image.

Based on the fourth RGB image, natural tooth region detector 112 detects a natural tooth region that is a region showing one or more natural teeth from a region showing one or more teeth within the fourth RGB image. Based on the fourth RGB image, dental plaque region detector 113 detects a dental plaque region that is a region with dental plaque from regions within the fourth RGB image. Image generator 114 generates a display image showing the dental plaque region. Specifically, image generator 114 generates a display image displaying the dental plaque region restricted to the natural tooth region. For instance, the display image is an image in which the dental plaque region is superimposed onto image data (the fourth RGB image).

Display 103 is a display device included in portable terminal 70, and displays the display image generated by image generator 114.

Hereinafter, the image generation processing (step S104 illustrated in FIG. 4) is described in detail with reference to FIGS. 6 to 14. FIG. 6 is a flowchart illustrating details of the image generation processing.

FIG. 7 illustrates an example of an RGB image (a fourth RGB image). The RGB image illustrated in FIG. 7 is an image showing natural teeth 201, artificial tooth 202, and gingiva 203 (gums). Artificial tooth 202 is, for example, a prosthesis made of metal, such as gold or silver. Natural tooth 201 is a natural tooth and is a portion excluding artificial tooth 202 from a tooth.

Frist, natural tooth region detector 112 detects a first natural tooth region using the RGB image (the fourth RGB image) (S201). Specifically, natural tooth region detector 112 detects, as the first natural tooth region, a region in the RGB image that satisfies both a first condition where a green pixel value (G) is greater than or equal to a first threshold and a second condition where each of the pairwise differences between a green pixel value (G), a red pixel value (R), and a blue pixel value (B) is less than a predetermined third threshold.

Here, when a natural tooth is irradiated with excitation light (blue light), the dentin of the tooth emits excitation fluorescence. The excitation fluorescence transmits through the enamel of the tooth. Thus, the natural tooth fluoresces green. Moreover, when being irradiated with blue light, a filling used to repair a carious tooth appears dark (has a low luminance) in an image captured by a camera, unlike in a state where the filling is irradiated with white light. Meanwhile, the natural tooth covered with enamel is bright (has a high luminance) in the image. Thus, based on the first condition, it is possible to identify the natural tooth region by extracting the green fluorescence, and exclude an artificial tooth region.

Moreover, specifically, the second condition is a condition that satisfies the following: (1) the absolute value (abs (R-G)) of the difference between the red pixel value (R) and the green pixel value (G) is less than threshold rg_th, (2) the absolute value (abs (G-B)) of the difference between the green pixel value (G) and the blue pixel value (B) is less than threshold gb_th, and (3) the absolute value (abs (B-R)) of the difference between the blue pixel value (B) and the red pixel value (R) is less than threshold br_th. It should be noted that threshold rg_th, threshold gb_th, and threshold br_th may be the same value or different values.

Moreover, in the first condition, a luminance value (Y) may be used instead of the green pixel value (G). The luminance value (Y) is calculated using the following Expression (3).$Y = 0.21 * R + 0.72 * G + 0.07 * B$

In Expression (3), Y denotes a luminance value, R denotes a red pixel value, G denotes a green pixel value, and B denotes a blue pixel value. As illustrated in Expression (3), the green pixel value accounts for a large proportion in the luminance value. Thus, even using the luminance value, it is possible to perform similar detection as when the green pixel value is used.

It should be noted that in the example illustrated herein, both the first condition and the second condition are used. However, only one of the first condition or the second condition may be used. For instance, only the first condition may be used.

FIG. 8 illustrates an example of detected first natural tooth region 211. It should be noted that for the sake of explanation, FIG. 8 illustrates shapes of teeth and so forth in addition to first natural tooth region 211. However, detected information need not include the shapes of the teeth and so forth. The same applies to FIGS. 9 to 13.

As illustrated in FIG. 8, a region including a region showing natural teeth 201 and a region showing gingiva 203 is detected as first natural tooth region 211. That is, first natural tooth region 211 does not include artificial tooth 202.

It should be noted that in the example illustrated herein, first natural tooth region 211 includes the entire region showing gingiva 203. However, according to the imaging situation, (i) a case where first natural tooth region 211 includes only a portion of the region showing gingiva 203 or (ii) a case where first natural tooth region 211 does not include the region showing gingiva 203 may occur.

Next, natural tooth region detector 112 generates an HSV image by converting the color space of the RGB image (the fourth RGB image) into an HSV space (S202). Then, natural tooth region detector 112 detects a gingival region by using the HSV image (S203). Specifically, natural tooth region detector 112 detects, as the gingival region, a region where hue H falls within a predetermined range.

FIG. 9 illustrates an example of detected gingival region 212. As illustrated in FIG. 9, the region showing gingiva 203 is detected as gingival region 212. It should be noted that although a gum region is detected in the example illustrated herein, lips and the like, as well as the gums may be detected as gingival region 212.

Then, natural tooth region detector 112 determines, as second natural tooth region 213, a region obtained by excluding gingival region 212 from first natural tooth region 211 (S204). FIG. 10 illustrates an example of second natural tooth region 213. As illustrated in FIG. 10, second natural tooth region 213 includes the region showing natural teeth 201, and does not include the region showing artificial tooth 202 or the region showing gingiva 203.

Then, natural tooth region detector 112 determines a third natural tooth region by excluding a small region from second natural tooth region 213 (S205). Here, the small region is a region with an area smaller than a predetermined value. Thus, it is possible to suppress falsely determining as the natural tooth region, for example, a region with a high luminance due to the reflected light or the like in a region (for example, the gingival region) other than the natural tooth region.

Then, natural tooth region detector 112 determines a fourth natural tooth region by extending the third natural tooth region (S206). Specifically, natural tooth region detector 112 determines, as the fourth natural tooth region, a region obtained by extending the boundary of the third natural tooth region outward by a predetermined amount. Thus, it is possible to add, for example, the boundary between the teeth and gums to the natural tooth region, which can prevent the dental plaque region from being excessively omitted from display.

Then, dental plaque region detector 113 detects a first dental plaque region (S207). Specifically, as the first dental plaque region, dental plaque region detector 113 detects a region where one or more pixels are located that satisfy at least one of: (i) the saturation falls within a first predetermined range (for example, at least 30 and at most 80 in the 8-bit representation), (ii) the hue falls within a second predetermined range (for example, at least 140 and at most 170 in the 8-bit representation), or (iii) the value (brightness) falls within a third predetermined range (for example, at least 100 and at most 180 in the 8-bit representation), the one or more pixels being included in the pixels of the HSV image generated in step S202. It should be noted that the first predetermined range, the second predetermined range, and the third predetermined range may be identified by comparing the actual dental plaque region, the actual tooth region, and HSV image, and are not limited to the numerical value ranges described above.

It should be noted that the value ranges of saturation, hue, and value (brightness) can be determined by comparing with the dyeing degree of dental plaque dyed by an applied dental plaque disclosing agent.

FIG. 11 illustrates an example of detected first dental plaque region 214. As illustrated in FIG. 11, a region with dental plaque is detected as first dental plaque region 214. Moreover, in this example, first dental plaque region 214 includes the region showing artificial tooth 202. That is, the region showing artificial tooth 202 is falsely detected as the dental plaque region.

It should be noted that although in the example illustrated herein, information indicating whether or not dental plaque is present as first dental plaque region 214 (binary) is detected, the amount of dental plaque (multiple values) may be detected. For instance, by comparing the intensity of red fluorescence per unit area of first dental plaque region 214, it is possible to detect the accumulation level (concentration or density) of a fluorescent substance. For instance, dental plaque region detector 113 may detect the accumulation level based on the value of value V of the HSV image of first dental plaque region 214. Moreover, dental plaque region detector 113 may perform gradation display according to the accumulation level.

Then, image generator 114 generates a display image by extracting first dental plaque region 214 within the fourth natural tooth region (S208). Specifically, image generator 114 generates a mask image in which the fourth natural tooth region takes a value of 1 (white) and the other regions take a value of 0 (black). FIG. 12 illustrates an example of mask image 215. As illustrated in FIG. 12, in mask image 215, the region showing natural teeth 201 has a value of 1 (white), and the regions other than natural teeth 201 have a value of 0 (black).

Then, image generator 114 calculates a second dental plaque region by calculating a logical conjunction of mask image 215 and an image showing first dental plaque region 214 illustrated in FIG. 11. That is, as the second dental plaque region, image generator 114 extracts the portion of first dental plaque region 214 that is included in the fourth natural tooth region. In other words, image generator 114 determines the second dental plaque region by excluding, from first dental plaque region 214, one or more regions included in the region other than the fourth natural tooth region.

FIG. 13 illustrates an example of the second dental plaque region. As illustrated in FIG. 13, a region obtained by excluding the region showing artificial tooth 202 from first dental plaque region 214 is determined as second dental plaque region 216.

Then, image generator 114 generates a display image in which second dental plaque region 216 is superimposed onto the first RGB image (FIG. 7). FIG. 14 illustrates an example of the display image. As illustrated in FIG. 14, when information indicating the dental plaque region is displayed on the tooth image, the user can easily identify unbrushed areas or the like. Moreover, the above processing can suppress false detection of a prosthesis or the like as dental plaque.

It should be noted that the processing procedure illustrated in FIG. 6 is a mere example, and the present disclosure is limited to the procedure. For instance, the timing when the dental plaque detection processing (S207) is performed may be any timing prior to step S208. Moreover, it is not necessary to perform all processes illustrated in FIG. 6, and some processes need not be performed. For instance, at least some of the processes corresponding to steps S203 to S206 need not be performed.

Moreover, although in the example described above, an artificial tooth is made of metal, such as gold or silver, the artificial tooth may be made of, for example, ceramic or zirconia, and is not limited to metal. As described above, when a natural tooth is irradiated with excitation light (blue light), a natural tooth region is detected based on the fact that natural teeth fluoresce green. Accordingly, an artificial tooth made of a material other than metal can also be excluded from the natural tooth region in the same manner. Meanwhile, it is not required to exclude, using the above method, artificial teeth of every material from the natural tooth region. Using the above method, artificial teeth made of at least some materials (for example, metal) can be excluded from the natural tooth region. Accordingly, it is possible to realize the effect of suppressing false detection as compared with a case where the above method is not used.

It should be noted that in the above embodiment, as a nonlimiting example, intraoral camera 10 transmits the first RGB image to portable terminal 70, and portable terminal 70 then performs processing on the first RGB image. The first RGB image may be transmitted to cloud server 80, and cloud server 80 may then perform the above processing and transmit the second RGB image or the fourth RGB image, which is the processing result, to portable terminal 70. In this case, the first RGB image may be transmitted from intraoral camera 10 to cloud server 80 without via portable terminal 70 or may be transmitted from intraoral camera 10 to cloud server 80 via portable terminal 70.

As described above, the image processing method according to the embodiment is an image processing method that displays a dental plaque region based on an image (for example, a first RGB image) showing a tooth and dental plaque fluorescing in response to exposure to light including a blue-light wavelength range. The image processing method includes: detecting a natural tooth region from a region showing the tooth, based on the image (for example, S104 in FIG. 4, S201 to S206 in FIG. 6); detecting a dental plaque region based on the image (for example, S207 in FIG. 6); and displaying the dental plaque region restricted to the natural tooth region (for example, S105 in FIG. 4). Thus, the image processing method can suppress false detection of a prosthesis or the like as dental plaque. Accordingly, the image processing method can improve the detection accuracy of dental plaque.

For instance, in the image processing method, detecting of the natural tooth region includes detecting a first region (for example, the first natural tooth region) that is (i) a region with a luminance value greater than or equal to a predetermined first threshold in an all-pixel region of the image or (ii) a region with a green pixel value greater than or equal to a predetermined second threshold in the all-pixel region of the image, to detect the natural tooth region based on the first region (for example, S201 in FIG. 6). Thus, the image processing method can detect the natural tooth region with high accuracy by using the luminance value or the green pixel value.

For instance, in the image processing method, detecting of the natural tooth region includes detecting, as the natural tooth region, a region which is included in the first region and in which each of the pairwise differences between the green pixel value, the red pixel value, and the blue pixel value is less than a predetermined third threshold (for example, S201 in FIG. 6). Thus, the image processing method can detect the natural tooth region with high accuracy by using the pairwise differences between the green pixel value, the red pixel value, and the blue pixel value.

For instance, in the image processing method, detecting of the natural tooth region includes: detecting a gingival region based on the hue of the image (for example, S203 in FIG. 6); and detecting, as the natural tooth region, a region obtained by excluding the gingival region from the first region (for example, S204 in FIG. 6). Thus, the image processing method can suppress false detection of the gingival region as the natural tooth region, which, in turn, can improve the detection accuracy of the natural tooth region.

For instance, in the image processing method, detecting of the natural tooth region includes detecting, as the natural tooth region, a region obtained by excluding a region having an area smaller than a predetermined area from the first region (for example, S205 in FIG. 6). Thus, the image processing method can suppress falsely determining as the natural tooth region, for example, a region with a high luminance due to reflected light or the like in a portion other than the natural tooth region, which, in turn, can improve the detection accuracy of the natural tooth region.

For instance, in the image processing method, detecting of the natural tooth region includes detecting, as the natural tooth region, a region obtained by extending the boundary of the first region outward by a predetermined amount (for example, S206 in FIG. 6). Thus, by extending the natural tooth region, the image processing method can prevent the dental plaque region from being excessively omitted from display.

Moreover, an image processing device (for example, portable terminal 70) according to the embodiment is an image processing device that displays a dental plaque region based on an image (for example, a first RGB image) showing a tooth and dental plaque fluorescing in response to exposure to light including a blue-light wavelength range. The image processing device includes: natural tooth region detector 112 that detects a natural tooth region from a region showing the tooth, based on the image, dental plaque region detector 113 that detects a dental plaque region based on the image, and display 103 that displays the dental plaque region restricted to the natural tooth region. Thus, the image processing device can suppress false detection of a prosthesis or the like as dental plaque. Accordingly, the image processing device can improve the detection accuracy of dental plaque.

Although the intraoral camera system according to the embodiment of the present disclosure is described above, the present disclosure is not limited to the embodiment.

For instance, in the examples described above, intraoral camera 10 mainly intended for imaging teeth is used. However, intraoral camera 10 may be an oral care device including a camera. For instance, intraoral camera 10 may be, for example, a dental washer including a camera.

Moreover, the processing units included in the intraoral camera system according to the embodiment are typically implemented as LSIs, which are integrated circuits. Each processing unit may be integrated into an individual chip, or some or all of the processing units may be integrated into a single chip.

Moreover, circuit integration is not limited to an LSI, and may be realized by a dedicated circuit or a general-purpose processor. A field programmable gate array (FPGA) that can be programmed after LSI manufacturing, or a reconfigurable processor that can reconfigure the connection or settings of circuit cells inside the LSI may be used.

Moreover, in the embodiment described above, each element may be implemented as dedicated hardware or by executing a software program suitable for the element. Each element may be implemented by a program executer, such as a CPU or a processor, reading out and executing the software program stored in a recording medium, such as a hardware disk or semiconductor memory.

Moreover, the present disclosure may be implemented as an image processing method or the like performed by the intraoral camera system. Moreover, the present disclosure may be implemented as the intraoral camera, the portable terminal, or the cloud server included in the intraoral camera system.

Moreover, the division of functional blocks in a block diagram is merely an example. A plurality of functional blocks may be formed as a single functional block, a single functional block may be divided into a plurality of functional blocks, or some functions may be transferred to other functional blocks. Moreover, functions of a plurality of functional blocks having similar functions may be processed in parallel or in a time-division manner by a single piece of hardware or software.

Moreover, the order of the steps in each flowchart is presented for illustrative purposes to explain the present disclosure in detail, and the steps may be performed in a different order. Moreover, some of the steps may be performed with other steps simultaneously (in parallel).

The intraoral camera system and so forth according to one or more aspects are described above based on the embodiment. However, the present disclosure is not limited to the embodiment. The one or more aspects may encompass embodiments obtained by adding various modifications envisioned by those skilled in the art to the above embodiment, as well as embodiments created by combining elements from different embodiments, provided that such embodiments remain within the scope of the present disclosure.

### [Industrial Applicability]

The present disclosure is applicable to intraoral camera systems.

### [Reference Signs List]

- 10: intraoral camera
- 10a: head
- 10b: handle
- 10c: neck
- 12: imaging optical system
- 12a: light entry port
- 14: image sensor
- 16: lens
- 18: mirror
- 20: blue-light blocking filter
- 24: aperture
- 26A, 26B, 26C, 26D: LED
- 28: cover
- 30: composition adjustment mechanism
- 32: focus adjustment mechanism
- 34: case
- 36, 40: actuator
- 38: lens holder
- 50: central controller
- 54: LED controller
- 56: lens driver
- 58: wireless communication module
- 60: power supply controller
- 62: controller
- 64: memory
- 66: battery
- 68: coil
- 69: charger
- 70: portable terminal
- 72: touch screen
- 80: cloud server
- 90: position sensor
- 101: obtainer
- 102: generator
- 103: display
- 111: image processor
- 112: natural tooth region detector
- 113: dental plaque region detector
- 114: image generator
- 201: natural tooth
- 202: artificial tooth
- 203: gingiva
- 211: first natural tooth region
- 212: gingival region
- 213: second natural tooth region
- 214: first dental plaque region
- 215: mask image
- 216: second dental plaque region

## Claims

1. An image processing method for displaying a dental plaque region based on an image showing a tooth and dental plaque fluorescing in response to exposure to light including a blue-light wavelength range, the image processing method comprising:
detecting a natural tooth region from a region showing the tooth, based on the image;
detecting a dental plaque region based on the image; and
displaying the dental plaque region restricted to the natural tooth region.

2. The image processing method according to claim 1, wherein
the detecting of the natural tooth region includes:
detecting a first region that is (i) a region with a luminance value greater than or equal to a predetermined first threshold within an all-pixel region in the image or (ii) a region with a green pixel value greater than or equal to a predetermined second threshold within the all-pixel region in the image, to detect the natural tooth region based on the first region.

3. The image processing method according to claim 2, wherein
the detecting of the natural tooth region includes:
detecting, as the natural tooth region, a region which is included in the first region and in which each of pairwise differences between a green pixel value, a red pixel value, and a blue pixel value is less than a predetermined third threshold.

4. The image processing method according to claim 2 or 3, wherein
the detecting of the natural tooth region includes:
detecting a gingival region based on a hue of the image; and
detecting, as the natural tooth region, a region obtained by excluding the gingival region from the first region.

5. The image processing method according to claim 2 or 3, wherein
the detecting of the natural tooth region includes:
detecting, as the natural tooth region, a region obtained by excluding a region having an area smaller than a predetermined area from the first region.

6. The image processing method according to claim 2 or 3, wherein
the detecting of the natural tooth region includes:
detecting, as the natural tooth region, a region obtained by extending a boundary of the first region outward by a predetermined amount.

7. An image processing device that displays a dental plaque region based on an image showing a tooth and dental plaque fluorescing in response to exposure to light including a blue-light wavelength range, the image processing device comprising:
a natural tooth region detector that detects a natural tooth region from a region showing the tooth, based on the image;
a dental plaque region detector that detects a dental plaque region based on the image; and
a display that displays the dental plaque region restricted to the natural tooth region.

8. A program for causing a computer to execute the image processing method according to claim 1.
